# EUROPÄISCHE PATENTSCHRIFT

(11) **EP 1 242 390 B1**
(45) Veröffentlichungstag und Bekanntmachung des Hinweises auf die Patenterteilung: **11.08.2004**
(21) Anmeldenummer: 00983274.2
(22) Anmeldetag: 08.12.2000
(51) Int. Cl.: C07D 251/08

(54) **VERFAHREN ZUR HERSTELLUNG VON 1,3-DISUBSTITUIERTEN 2-NITROGUANIDINEN**
METHOD FOR PRODUCTION OF 1,3-DISUBSTITUTED 2-NITROGUANIDINES
PROCEDE DE PREPARATION DE 2-NITROGUANIDINES 1,3-DISUBSTITUEES

(30) Priorität: 21.12.1999 DE 19961604
(43) Veröffentlichungstag der Anmeldung: 25.09.2002
(73) Patentinhaber: Bayer CropScience AG, 40789 Monheim (DE)
(72) Erfinder: VAN LAAK, Kai, 51061 Köln (DE); SIRGES, Wolfram, 40597 Düsseldorf (DE); WOLLWEBER, Detlef, 42113 Wuppertal (DE)
(86) Internationale Anmeldenummer: PCT/EP2000/012495
(87) Internationale Veröffentlichungsnummer: WO 2001/046160

(56) Entgegenhaltungen:
- EP-A- 0 483 062
- WO-A-99/09009
- PATENT ABSTRACTS OF JAPAN vol. 1998, no. 11, 30. September 1998 (1998-09-30) & JP 10 147580 A (MITSUI CHEM INC), 2. Juni 1998 (1998-06-02)

## Beschreibung

Die vorliegende Erfindung betrifft ein neuartiges Verfahren zur Herstellung von 1,3-disubstituierten 2-Nitroguanidinen.

Aus EP-A-0 483 062 ist ein Verfahren zur Herstellung von 1,3-disubstituierten 2-Nitroguanidinen bekannt. Sie werden erhalten durch Hydrolyse entsprechender 2-Nitroimino-1,3,5-triazacyclohexanderivate. Die Hydrolyse wird bevorzugt in Gegenwart starker Mineralsäuren oder organischer Säuren durchgeführt.

Nachteilig bei diesem Verfahren sind die langen Reaktionszeiten und das Entstehen von Nebenprodukten, die eine aufwendige Reinigung der gewünschten Endprodukte erforderlich machen.

Darüber hinaus müssen beim Arbeiten in Gegenwart wässriger starker Säuren bekanntlich aufwendige Maßnahmen zum Schutz beispielsweise der Reaktoren gegen Korrosion getroffen werden.

Die Anmeldungen JP 03 291 267, JP 10067766 und JP10147580 betreffen ähnliche Verfahren.

Aufgabe der vorliegenden Erfindung war es, ein verbessertes Verfahren zur Herstellung von 1,3-disubstituierten 2-Nitroguanidinen bereitzustellen.

Gegenstand der vorliegenden Erfindung ist ein Verfahren zur Herstellung von Verbindungen der Formel (I) 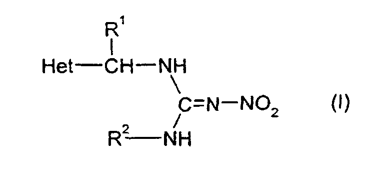 worin
- R¹: für Wasserstoff oder Alkyl steht,
- R²: für Wasserstoff, Alkyl, Cycloalkyl oder -CH₂R³ steht,
- R³: für Alkenyl, Alkinyl oder jeweils gegebenenfalls substituiertes Aryl oder Heteroaryl steht,
- Het: für einen unsubstituierten oder substituierten aromatischen oder nichtaromatischen, monocyclischen oder bicyclischen heterocyclischen Rest, bevorzugt aus der Reihe 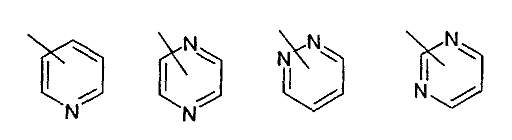 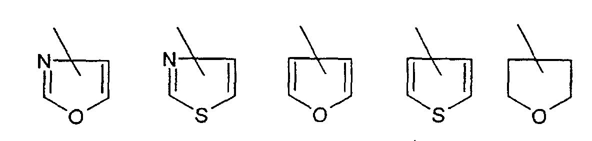 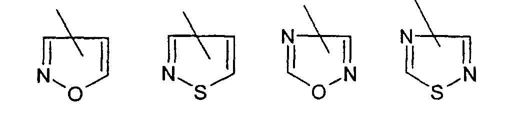 steht,
dadurch gekennzeichnet, dass man eine Verbindung der Formel (II) 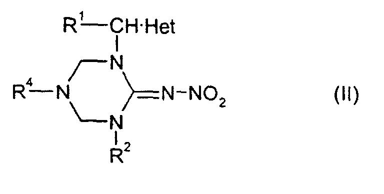 worin
- R¹, R² und Het: die oben angegebenen Bedeutungen haben und
- R⁴: für jeweils unsubsituiertes oder substituiertes Alkyl, Cycloalkyl, Aryl, Arylalkyl oder Heterocyclylalkyl steht,
gegebenenfalls in Gegenwart eines Verdünnungsmittels mit wasserfreiem Chlorwasserstoff oder mit einer oder mehreren Verbindungen, die Chlorwasserstoff entwickeln können umsetzt.

Die Verbindungen der Formel (I) können auch als Doppelbindungsisomere bezüglich der -N=C(2)-Bindung und in ihren tautomeren Formen (Formeln Ia, Ib) auftreten:

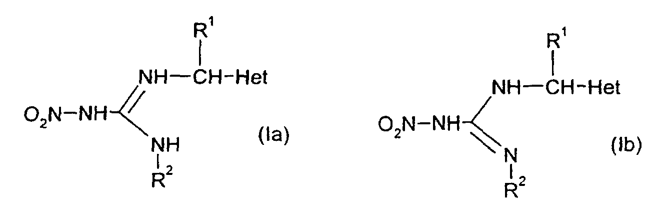

Formel (I) ist demnach so zu verstehen, dass sie auch die entsprechenden Doppelbindungsisomeren und die Formeln (Ia) und (Ib) einschließt.

Überraschenderweise liefert das erfindungsgemäße Verfahren selektiv und in hohen Ausbeuten die Endprodukte der Formel (I) nach kurzer Reaktionszeit unter milden Reaktionsbedingungen in reiner Form.

Verwendet man beispielsweise 1-(2-Chlorthiazol-5-ylmethyl)-2-nitro-imino-5-benzyl-3-inethyl-1,3,5-triazacyclohexan als Ausgangsstoff, so kann der Verlauf des erfindungsgemäßen Verfahrens durch das folgende Formelschema wiedergegeben werden:

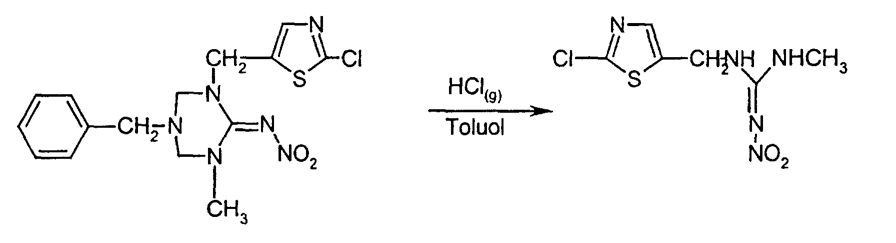

Die als Ausgangsstoffe für das erfindungsgemäße Verfahren benötigten Verbindungen sind durch die Formel (II) allgemein definiert.

Bevorzugte Substituenten bzw. Bereiche der in den oben und nachstehend erwähnten Formeln aufgeführten Reste werden im folgenden erläutert:
- R¹: steht bevorzugt für Wasserstoff oder C₁-C₄-Alkyl,
- R²: steht bevorzugt für Wasserstoff, C₁-C₆-Alkyl, C₃-C₆-Cycloalkyl oder -CH₂R³,
- R³: steht bevorzugt für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, für durch ein bis zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl, C₂-C₃-Alkenyl, C₂-C₃-Alkinyl, C₁-C₃-Alkoxy, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Halogen, Cyano und Nitro substituiertes 5-Thiazolyl; oder für durch ein bis vier (bevorzugt ein oder zwei) Reste aus der Gruppe C₁-C₃-Halogenalkyl mit 1 bis 7 Halogenatomen, Cyclopropyl, Halogencyclopropyl, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy und Halogen substituiertes 3-Pyridyl,
- Het: steht bevorzugt für einen unsubstituierten oder substituierten aromatischen oder nichtaromatischen, monocyclischen oder bicyclischen heterocyclischen Rest, bevorzugt aus der Reihe 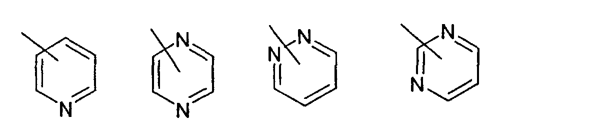 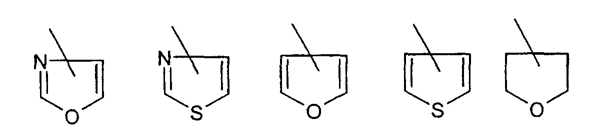 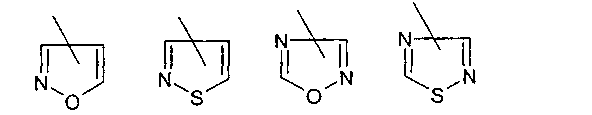 der, auch in Abhängigkeit von der Art des Heterocyclus, ein oder zwei Substituenten aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy und Halogen enthalten kann.
- R⁴: steht bevorzugt für C₁-C₁₀-Alkyl, C₃-C₆-Cycloalkyl, durch 1 bis 6 Reste aus der Gruppe Halogen, Hydroxy, C₁-C₄-Alkoxy, C₁-C₄-Halogenalkoxy mit 1 bis 9 Halogenatomen, Di-( C₁-C₄-Alkyl)-amino und C₁-C₅-Alkoxycarbonyl substituiertes C₁-C₁₀-Alkyl, durch 1 bis 4 Reste aus der Reihe C₁-C₄-Alkyl und Halogen substituiertes C₃-C₆-Cycloalkyl, Phenyl, Benzyl, oder durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro oder Cyano substituiertes Phenyl oder Benzyl, oder Heterocyclylmethyl, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff, Schwefel (insbesondere Furyl, Tetrahydrofuryl, Thienyl oder Pyridyl).
- R¹: steht besonders bevorzugt für Wasserstoff, Methyl, Ethyl, n- oder i-Propyl,
- R²: steht besonders bevorzugt für Wasserstoff, Methyl, Ethyl, n-Propyl, i-Propyl oder n-Butyl, Cyclopropyl, Cyclopentyl, Cyclohexyl oder für -CH₂R³,
- R³: steht besonders bevorzugt für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, für durch ein bis zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl, C₂-C₃-Alkenyl, C₂-C₃-Alkinyl, C₁-C₃-Alkoxy, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Halogen, Cyan oder Nitro substituiertes 5-Thiazolyl; oder für durch ein bis zwei (bevorzugt einen) Reste aus der Gruppe C₁-C₃-Halogenalkyl mit 1 bis 7 Halogenatomen, Cyclopropyl, Halogencyclopropyl, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy oder Halogen substituiertes 3-Pyridyl.
- Het: steht besonders bevorzugt für einen unsubstituierten oder einfach oder zweifach (bevorzugt einfach) substituierten heterocyclischen Rest aus der Reihe 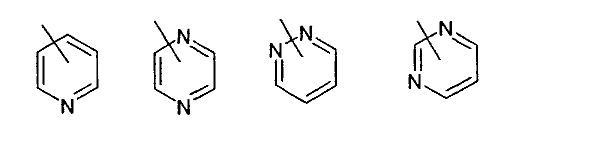 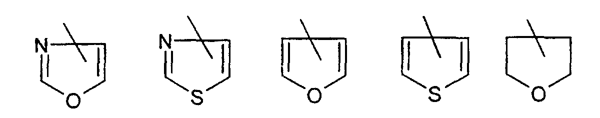 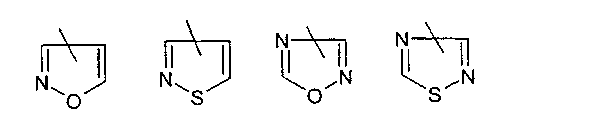 insbesondere aus der Reihe 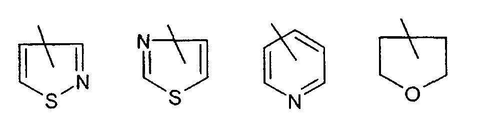 wobei die Substituenten aus der Reihe Fluor, Chlor, Brom, Methyl, Ethyl, Methoxy und Ethoxy ausgewählt sind.
- R⁴: steht besonders bevorzugt für gegebenenfalls durch Halogen (insbesondere Fluor oder Chlor) substituiertes C₁-C₄-Alkyl oder für jeweils gegebenenfalls durch Halogen (insbesondere Fluor oder Chlor) oder C₁-C₄-Alkyl substituiertes C₃-C₆-Cycloalkyl, Phenyl, Phenyl-C₁-C₄-alkyl oder Heterocyclylmethyl, wobei Heterocyclyl für einen ungesättigten oder gesättigten 5- oder 6-gliedrigen Heterocyclus mit einem oder mehreren Heteroatomen aus der Reihe Stickstoff, Sauerstoff und Schwefel (insbesondere für Thienyl, Pyridyl, Furyl oder Tetrahydrofuryl).
- R¹: steht ganz besonders bevorzugt für Wasserstoff, Methyl oder Ethyl,
- R²: steht ganz besonders bevorzugt für Wasserstoff, Methyl, Ethyl, n-Propyl, i-Propyl, n-Butyl, Cyclopropyl, Cyclopentyl, Cyclohexyl oder für einen Rest -CH₂R³,
- R³: steht ganz besonders bevorzugt für C₂-C₃-Alkenyl, C₂-C₃-Alkinyl, Phenyl, Cyanophenyl, Nitrophenyl, Halogenphenyl mit 1 bis 3 Halogenatomen, für durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, für jeweils durch ein oder zwei (bevorzugt einen) Substituenten aus der Gruppe C₁-C₃-Alkyl, C₁-C₃-Halogenalkyl mit 1 bis 7 Halogenatomen, C₁-C₃-Alkoxy, C₁-C₃-Halogenalkoxy mit 1 bis 7 Halogenatomen, C₁-C₃-Alkylthio, C₁-C₃-Halogenalkylthio mit 1 bis 7 Halogenatomen, Halogen, Cyano oder Nitro substituiertes 5-Thiazolyl oder 3-Pyridyl,
- R⁴: steht ganz besonders bevorzugt für C₁-C₄-Alkyl, Cyclopropyl, Cyclopentyl oder Cyclohexyl durch Halogen substituiertes C₁-C₄-Alkyl, für jeweils durch 1 oder 2 Reste aus der Reihe Methyl, Ethyl, Fluor und Chlor substituiertes C₃-C₆-Cycloalkyl, für Phenyl, Benzyl, oder für jeweils durch 1 oder 2 Ringsubstituenten aus der Gruppe Methyl, Ethyl, Fluor und Chlor substituiertes Phenyl, Benzyl, Furylmethyl, Tetrahydrofurylmethyl, Thienylmethyl oder Pyridylmethyl.
- Het: steht ganz besonders bevorzugt für jeweils unsubstituiertes oder einfach oder zweifach (insbesondere einfach) substituiertes Thiazolyl, Pyridyl oder Tetrahydrofuranyl, wobei die Substituenten aus der Reihe Fluor, Chlor, Methyl und Methoxy ausgewählt sind.

In den Definitionen steht Halogen(atome), wenn nichts anderes angegeben ist, für F, Cl, Br, I, bevorzugt für F, Cl, Br, besonders bevorzugt für F, Cl.
- R¹: steht ganz besonders hervorgehoben für Wasserstoff, Methyl oder Ethyl, hervorgehoben für Wasserstoff.
- R²: steht ganz besonders hervorgehoben für Wasserstoff, Methyl, Ethyl, n-Propyl, Cyclopropyl, Cyclopentyl, Allyl, Propargyl oder p-Chlorbenzyl, hervorgehoben für Methyl.
- R⁴: steht ganz besonders hervorgehoben für Methyl, Ethyl, n-Propyl, Cyclopropyl, Cyclopentyl, Cyclohexyl, Phenyl, Benzyl oder Tetrahydrofurylmethyl.
- Het: steht ganz besonders bevorzugt für einen der Reste

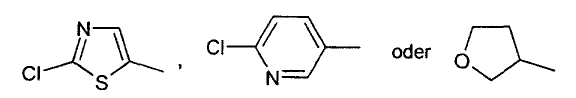

Besonders hervorgehoben seien als Ausgangsstoffe für das erfindungsgemäße Verfahren Verbindungen der Formel (IIa) 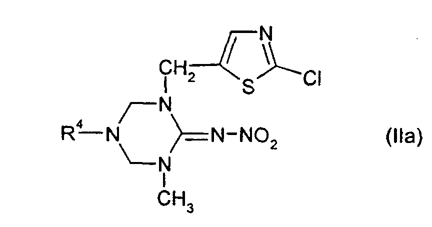 worin
- R⁴: für Methyl, Ethyl, Cyclopropyl, Cyclopentyl, Benzyl oder Tetrahydrofurylmethyl steht, wobei unter diesen wiederum Methyl, Benzyl und Tetrahydrofurylmethyl bevorzugt sind.

Besonders hervorgehoben seien als Ausgangsstoffe für das erfindungsgemäße Verfahren Verbindungen der Formel (IIb) und (IIc) 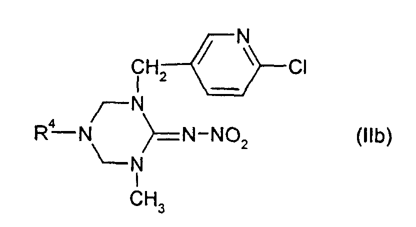 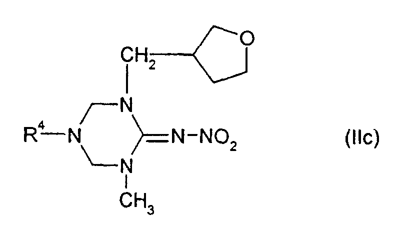 worin
- R⁴: die oben für die Verbindungen der Formel (IIa) angegebenen Bedeutungen hat.

Als Endprodukte des erfindungsgemäßen Verfahrens erhält man bei Verwendung der Verbindung der Formel (IIa) die folgende Verbindung 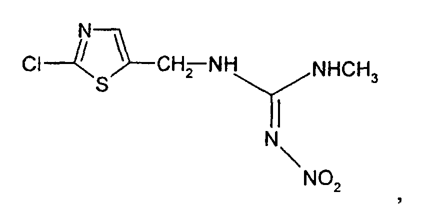 bei Verwendung der Verbindung der Formel (IIb) die folgende Verbindung 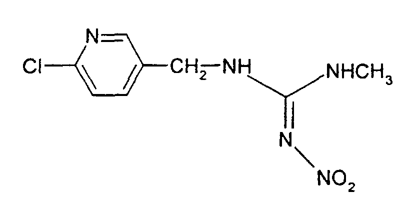 und bei Verwendung der Verbindung der Formel (IIc) die folgende Verbindung

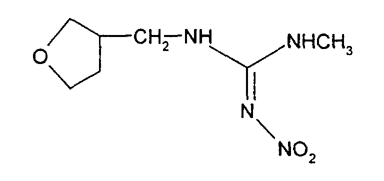

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Unter der Bezeichnung Alkyl sind dabei auch die verzweigten Isomere, z.B. t-Butyl für C₄-Alkyl, zu verstehen.

Bevorzugt werden diejenigen Verbindungen der Formel (II) in das erfindungsgemäße Verfahren eingesetzt, in welchen eine Kombination der vorstehend als bevorzugt auf geführten Bedeutungen vorliegt.

Besonders bevorzugt werden diejenigen Verbindungen der Formel (II) in das erfindungsgemäße Verfahren eingesetzt, in welchen eine Kombination der vorstehend als besonders bevorzugt aufgeführten Bedeutungen vorliegt.

Ganz besonders bevorzugt werden diejenigen Verbindungen der Formel (II) in das erfindungsgemäße Verfahren eingesetzt, in welchen eine Kombination der vorstehend als ganz besonders bevorzugt aufgeführten Bedeutungen vorliegt.

Die Ausgangsstoffe der Formel (II) sind bekannt oder können nach bekannten Verfahren hergestellt werden (vgl. EP-A-0 483 062, JP-03 291267, EP-A-0 483 055, EP-A-0 428 941, EP-A-0 386 565, WO 98/42690).

Bei der Durchführung des erfindungsgemäßen Verfahrens werden die Verbindungen der Formel (II) mit wasserfreiem Chlorwasserstoff oder mit Verbindungen umgesetzt, die mit protischen Lösungsmitteln, insbesondere mit Alkoholen oder Carbonsäuren Chlorwasserstoff entwickeln können.

Zu den Verbindungen, die Chlorwasserstoff entwickeln können, gehören beispielsweise Säurechloride, insbesondere Verbindungen der Formel (III) (Gruppe A): 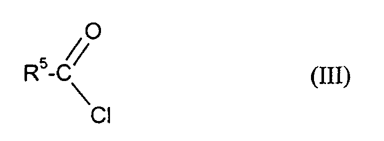 worin
- R⁵: für jeweils gegebenenfalls substituiertes Alkyl, Cycloalkyl, Aryl, Arylalkyl oder Heterocyclylalkyl steht.
- R⁵: steht bevorzugt für C₁-C₁₀-Alkyl, C₃-C₁₀-Cycloalkyl, Aryl-C₁-C₄-alkyl, insbesondere Phenyl-C₁-C₄-alkyl, jeweils gegebenenfalls einfach oder mehrfach substituiert, wobei als Substituenten OH, SH, Halogen, C₁-C₆-Alkoxy, C₁-C₆-Alkylthio, C₁-C₆-Alkyl und Aryl, insbesondere Phenyl in Frage kommen, oder für Heterocyclylmethyl, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff, Schwefel insbesondere Furyl, Tetrahydrofuryl, Thienyl oder Pyridyl.

Hierin steht Halogen(atome) bevorzugt für F, Cl, Br, I, insbesondere für F, Cl, Br und hervorgehoben für F, Cl.
- R⁵: steht besonders bevorzugt für C₁-C₄-Alkyl, C₃-C₆-Cycloalk-yl, Benzyl oder Phenylethyl, jeweils gegebenenfalls einfach bis fünffach (bevorzugt einfach bis dreifach, besonders bevorzugt einfach oder zweifach) substituiert, wobei als Substituenten, OH, Cl, Br, F, C₁-C₄-Alkoxy, C₁-C₄-Alkylthio, C₁-C₄-Alkyl und Aryl, insbesondere Phenyl in Frage kommen, oder für Heterocyclylmethyl, wobei Heterocyclyl insbesondere für Furyl, Tetrahydrofuryl, Thienyl oder Pyridyl steht.
- R⁵: steht ganz besonders bevorzugt für den jeweiligen Rest R⁴ der umzusetzenden Verbindung der Formel (II) oder einen der Reste aus der Reihe Benzyl, HO-CH₂-CH₂-, n-Hexyl oder Cyclohexyl.

Die Verbindungen der Formel (III) sind bekannt und im Handel erhältlich oder können leicht nach bekannten Verfahren hergestellt werden.

Zu den Verbindungen, die Chlorwasserstoff entwickeln können, gehören ferner (Gruppe B):
reaktive Nichtmetall- und Metallchloride sowie reaktive Nichtmetall- und Metalloxychloride, bevorzugt Bortrichlorid, Aluminiumtrichlorid, Siliciumtetrachlorid, Oxalylchlorid, Trichlorsilan, Phosphortrichlorid, Phosphorpentachlorid, Phosphoroxychlorid, Schwefeldichlorid, Titantetrachlorid, Titantrichlorid, Vanadiumtrichlorid, Vanadium(V)-oxytrichlorid, Thionylchlorid und Sulfurylchlorid, besonders bevorzugt Aluminiumchlorid, Phosphortrichlorid, Phosphorpentachlorid, Phosphoroxychlorid, Thionylchlorid, Sulfurylchlorid und Oxalylchlorid, ganz besonders bevorzugt Thionylchlorid, Sulfurylchlorid, Phosphoroxychlorid und Oxalylchlorid.

Die Verbindungen der Gruppe B sind bekannte Verbindungen, die als solche im Handel erhältlich sind oder sich in bekannter Weise herstellen lassen.

Das erfindungsgemäße Verfahren wird gegebenenfalls in Gegenwart eines Verdünnungsmittels durchgeführt.

Geeignete Verdünnungsmittel bei der Verwendung von Chlorwasserstoff sind organische Lösungsmittel, polare protische wie Methanol, Ethanol, n-Propanol, i-Propanol, n-Butanol oder i-Butanol, ferner polare aprotische Lösungsmittel, beispielsweise Aceton, Acetonitril und Essigester (z.B. Essigsäureethylester), Ether und cyclische Ether, wie Diethylether, Diisobutylether, THF, Dioxan, oder unpolare, aprotische Lösungsmittel wie Kohlenwasserstoffe beispielsweise Benzol, Toluol oder Xylol, halogenierte Kohlenwasserstoffe, wie Methylenchlorid, Chloroform, Tetrachlorkohlenstoff, Chlorbenzol oder o-Dichlorbenzol.

Es ist auch möglich, Gemische der genannten Verdünnungsmittel einzusetzen.

Als Verdünnungsmittel bei Verwendung von Verbindungen der Gruppe (A) oder (B) eignen sich polare, protische Lösungsmittel beispielsweise Alkohole oder Carbonsäuren.

Besonders geeignet sind Alkohole, insbesondere Methanol, Ethanol, n-Propanol, i-Propanol, n-Butanol, i-Butanol.

Es kann von Vorteil sein, der Reaktionsmischung ein weiteres Verdünnungsmittel zuzusetzen. Als solche kommen in Frage Ether, beispielsweise Dibutylether, THF, Dioxan, Glykoldimethylether oder Diglykoldimethylether, weiterhin Kohlenwasserstoffe, wie Benzol, Toluol oder Xylol, halogenierte Kohlenwasserstoffe, wie Methylenchlorid, Chloroform, Tetrachlorkohlenstoff, Chlorbenzol oder o-Dichlorbenzol, Nitrile wie Acetonitril, Carbonsäureester wie Ethylacetat oder auch Ketone wie Aceton oder Methylisopropylketon.

Es können auch Gemische der genannten Verdünnungsmittel eingesetzt werden.

Das erfindungsgemäße Verfahren wird im allgemeinen bei Temperaturen zwischen 0°C und 200°C, vorzugsweise zwischen 40°C und 150°C, durchgeführt.

Vorzugsweise wird unter Normaldruck gearbeitet, insbesondere im Falle niedrigsiedender Verdünnungsmittel kann gegebenenfalls auch unter erhöhtem Druck gearbeitet werden.

Der wasserfreie Chlorwasserstoff und die Verbindungen der Formel (III) bzw. der Gruppe B werden im allgemeinen in einem molaren Verhältnis von 0,5:1 bis 10:1, vorzugsweise 1:1 1 bis 5: 1, bezogen auf die Ausgangsverbindung der Formel (II), eingesetzt.

Im allgemeinen wird die Umsetzung so durchgeführt, dass man den Ausgangsstoff der Formel (II), und den Chlorwasserstoff bzw. die Verbindung der Formel (III) oder (IV) gegebenenfalls in einem Verdünnungsmittel und gegebenenfalls im Lösungsmittel auf die gewünschte Temperatur erhitzt. Es ist auch möglich, den Chlorwasserstoff oder die Verbindung der Formel (III) oder der Gruppe B im Verlauf der Reaktion sukzessive einzudosieren.

Zur Aufarbeitung wird nach dem Abkühlen gegebenenfalls mit Wasser versetzt und das Endprodukt gegebenenfalls nach Einengen des Gemischs beispielsweise durch Filtration oder Extraktion isoliert.

Bevorzugt wird die Reaktion in einem Verdünnungsmittel durchgeführt, aus welchem beim Abkühlen der Reaktionsmischung das Endprodukt direkt auskristallisiert und in einfacher Weise beispielsweise durch Filtration isoliert werden kann. Als Verdünnungsmittel kommen hierfür Alkohole in Frage, insbesondere Methanol, Ethanol, Propanol, i-Propanol, Isobutanol, n-Butanol, sec-Butanol.

Es ist auch möglich, eine wasserfreie Aufarbeitung des Reaktionsgemisches durchzuführen, indem man nach beendeter Reaktion gegebenenfalls das Verdünnungsmittel und gegebenenfalls Lösungsmittel abdestilliert und den verbleibenden Rückstand mit einem geeigneten Extraktionsmittel extrahiert. Als Extraktionsmittel kommen grundsätzlich alle gegenüber den Endprodukten inerten Lösungsmittel in Frage, in denen die Endprodukte ausreichend löslich sind.

Hierzu gehören beispielsweise aliphatische Kohlenwasserstoffe wie n-Pentan, n-Hexan, Cyclohexan, halogenierte aliphatische Kohlenwasserstoffe wie Methylenchlorid oder Chloroform, aromatische Kohlenwasserstoffe wie Benzol, Toluol oder Xylol, halogenierte aromatische Kohlenwasserstoffe wie Chlorbenzol oder o-Dichlorbenzol oder auch Ether wie beispielsweise Methyl-tert.-butylether.

Die Endprodukte kristallisieren, gegebenenfalls nach Einengen des Extraktionsmittels, aus und können durch Filtration isoliert werden, oder das Extraktionsmittel wird vollständig oder nahezu vollständig entfernt und der Rückstand, falls nötig, beispielsweise durch Umkristallisation gereinigt.

Die erfindungsgemäß hergestellten Verbindungen der Formel (I) sind wertvolle Wirkstoffe in der Schädlingsbekämpfung. Insbesondere sind die Verbindungen der Formel (I) geeignet zur Bekämpfung von Insekten und Spinnentieren, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen (siehe z.B. EP-A-0 376 279, EP-A-0 375 907, EP-A-0 383 091).

### Beispiele

### Beispiel 1

### Herstellung von 1-(2-Chlorthiazol-5-ylmethyl)-2-nitro-3-methylguanidin

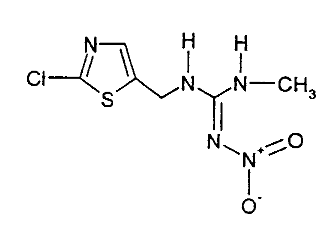

a) 19.2g 1-(2-Chlorthiazol-5-ylmethyl)-2-nitro-imino-5-benzyl-3-methyl-1,3,5-triazacyclohexan 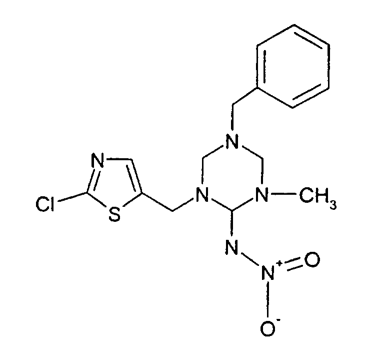 werden in 100 ml wasserfreiem Toluol gelöst und bei 65°C trockenes HCI-Gas eingeleitet. Es wird fünf Stunden bei 65°C nachgerührt und das Produkt durch Filtration isoliert. Das Kristallisat wird anschließend getrocknet.
   Ausbeute: 16.2 g, Reinheit (HPLC): 75 %, Selektivität: 99 %
   Das Rohprodukt wird mit 50 ml Butanol bei 50°C gerührt, der Feststoff bei 25°C filtriert und getrocknet.
   Ausbeute: 10.5 g , Reinheit (HPLC): 98 %
   Das Produkt ist nach seinen chromatographischen und spektroskopischen Daten identisch mit einer auf anderem Weg erhaltenen, authentischen Probe 1-(2-Chlorthiazol-5-ylmethyl)-2-nitro-3-methylguanidin.
b) 19.2g 1-(2-Chlorthiazol-5-ylmethyl)-2-nitro-imino-5-benzyl-3-methyl-1,3,5-triazacyclohexan werden in 100 ml wasserfreiem Aceton gelöst und bei 20°C trockenes HCI-Gas eingeleitet. Es wird fünf Stunden bei 20°C nachgerührt und das Produkt durch Filtration isoliert. Das Kristallisat wird anschließend getrocknet.
   Ausbeute: 19.8 g, Reinheit (HPLC): 62 %, Selektivität: 99 %
c) 19.2g 1-(2-Chlorthiazol-5-ylmethyl)-2-nitro-imino-5-benzyl-3-methyl-1,3,5-triazacyclohexan werden in 100 ml wasserfreiem Essigsäureethylester gelöst und bei 65°C trockenes HCI-Gas eingeleitet. Es wird fünf Stunden bei 65°C nachgerührt und das Produkt durch Filtration isoliert. Das Kristallisat wird anschließend getrocknet.
   Ausbeute: 16.5 g, Reinheit (HPLC): 75 %, Selektivität: 99 %
d) 19.2g 1-(2-Chlorthiazol-5-ylmethyl)-2-nitro-imino-5-benzyl-3-methyl-1,3,5-triazacyclohexan werden in 100 ml wasserfreiem Methanol gelöst und bei 20°C trockenes HCl-Gas eingeleitet. Es wird eine Stunde lang bei 20°C nachgerührt und das Produkt durch Filtration isoliert. Das Kristallisat wird anschließend getrocknet.
   Ausbeute: 14.2 g, Reinheit (HPLC): 79 %, Selektivität: 91 %
e) 19.2 g 1-(2-Chlorthiazol-5-ylmethyl)-2-nitro-imino-5-benzyl-3-methyl-1,3,5-triazacyclohexan werden in 100 ml wasserfreiem Methanol gelöst. Bei 40°C werden 5.1 g Acetylchlorid in 20 min zugetropft und dreieinhalb Stunden bei 40°C nachgerührt. Die erhaltene Suspension wird auf 0-5°C gekühlt und das Produkt durch Filtration isoliert. Das Kristallisat wird anschließend getrocknet.
   Ausbeute: 11.1 g, Reinheit (HPLC): 99 %
f) 19.2 g 1-(2-Chlorthiazol-5-ylmethyl)-2-nitro-imino-5-benzyl-3-methyl-1,3,5-triazacyclohexan werden in 100 ml wasserfreiem Methanol gelöst. Bei 25°C werden 7.7 g Thionylchlorid in 15 min zugetropft und drei Stunden bei dieser Temperatur nachgerührt. Die erhaltene Suspension wird auf 0-5°C gekühlt und das Produkt durch Filtration isoliert. Das Kristallisat wird anschließend getrocknet.
   Ausbeute 10.8 g, Reinheit (HPLC): 98 %
g) 19.2 g 1-(2-Chlorthiazol-5-ylmethyl)-2-nitro-imino-5-benzyl-3-methyl-1,3,5-triazacyclohexan werden in 100 ml wasserfreiem Methanol gelöst. Bei 25°C werden 8.3 g Oxalylchlorid in 25 min zugetropft und fünf Stunden bei 40°C nachgerührt. Die erhaltene Suspension wird auf 0-5°C gekühlt und das Produkt durch Filtration isoliert. Das Kristallisat wird anschließend getrocknet.
   Ausbeute 10.4 g, Reinheit (HPLC): 98 %
h) 19.2 g 1-(2-Chlorthiazol-5-ylmethyl)-2-nitro-imino-5-benzyl-3-methyl-1,3,5-triazacyclohexan werden in 100 ml wasserfreiem Methanol gelöst. Bei 25°C werden 6.3 g Chlorameisensäuremethylester in 10 min zugetropft und fünf Stunden bei 40°C nachgerührt. Die erhaltene Suspension wird auf 0-5°C gekühlt und das Produkt durch Filtration isoliert. Das Kristallisat wird anschließend getrocknet.
   Ausbeute 10.9 g, Reinheit (HPLC): 98 %
i) 19.2 g 1-(2-Chlorthiazol-5-ylmethyl)-2-nitro-imino-5-benzyl-3-methyl-1,3,5-triazacyclohexan werden in 100 ml wasserfreiem Methanol gelöst. Bei 25°C werden 7.3 g Chloracetylchlorid in 10 min zugetropft und fünf Stunden bei 40°C nachgerührt. Die erhaltene Suspension wird auf 0-5°C gekühlt und das Produkt durch Filtration isoliert. Das Kristallisat wird anschließend getrocknet.
   Ausbeute 10.9 g, Reinheit (HPLC): 98 %
j) 19.2 g 1-(2-Chlorthiazol-5-ylmethyl)-2-nitro-imino-5-benzyl-3-methyl-1,3,5-triazacyclohexan werden in 100 ml wasserfreiem Methanol gelöst. Bei 25°C werden 10 g Phosphoroxychlorid in 30 min zugetropft und ein Stunden bei 40°C nachgerührt. Die erhaltene Suspension wird auf 0-5°C gekühlt und das Produkt durch Filtration isoliert. Das Kristallisat wird anschließend getrocknet.
   Ausbeute: 9,1 g, Reinheit: 97,1 %

Analog können auch die in der folgenden Tabelle aufgeführten Verbindungen der Formel (I) erhalten werden:

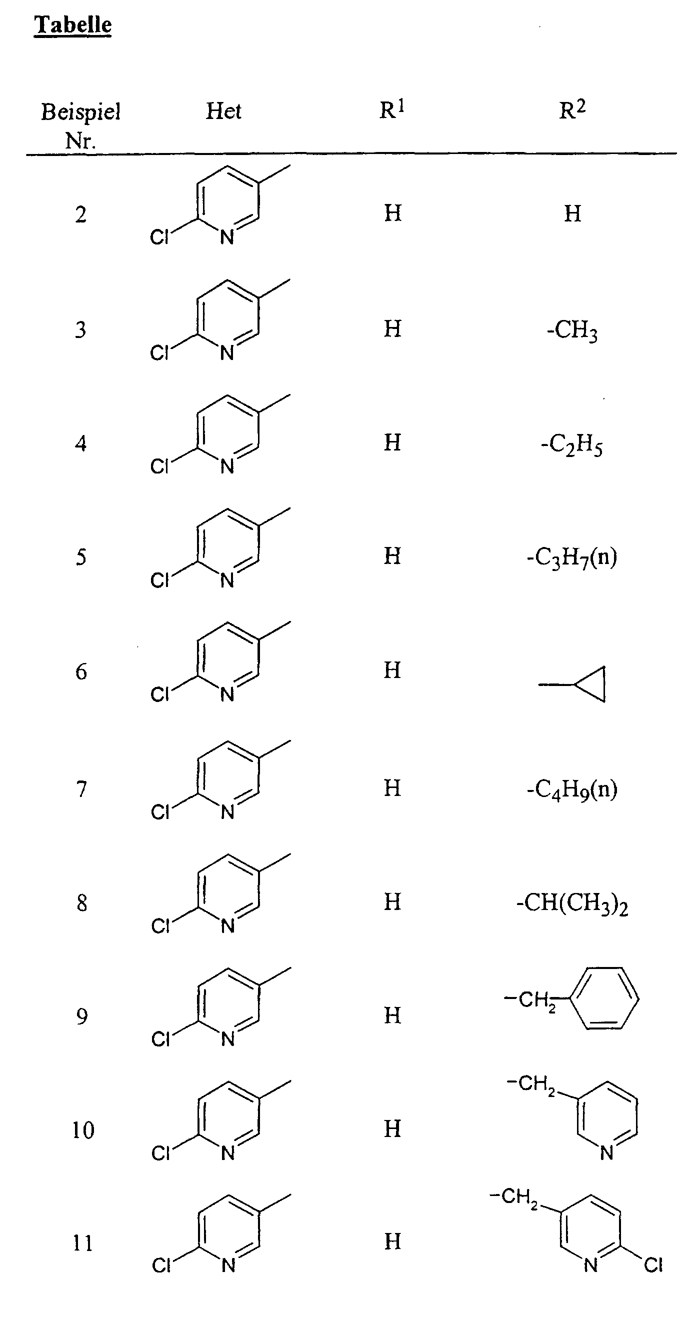

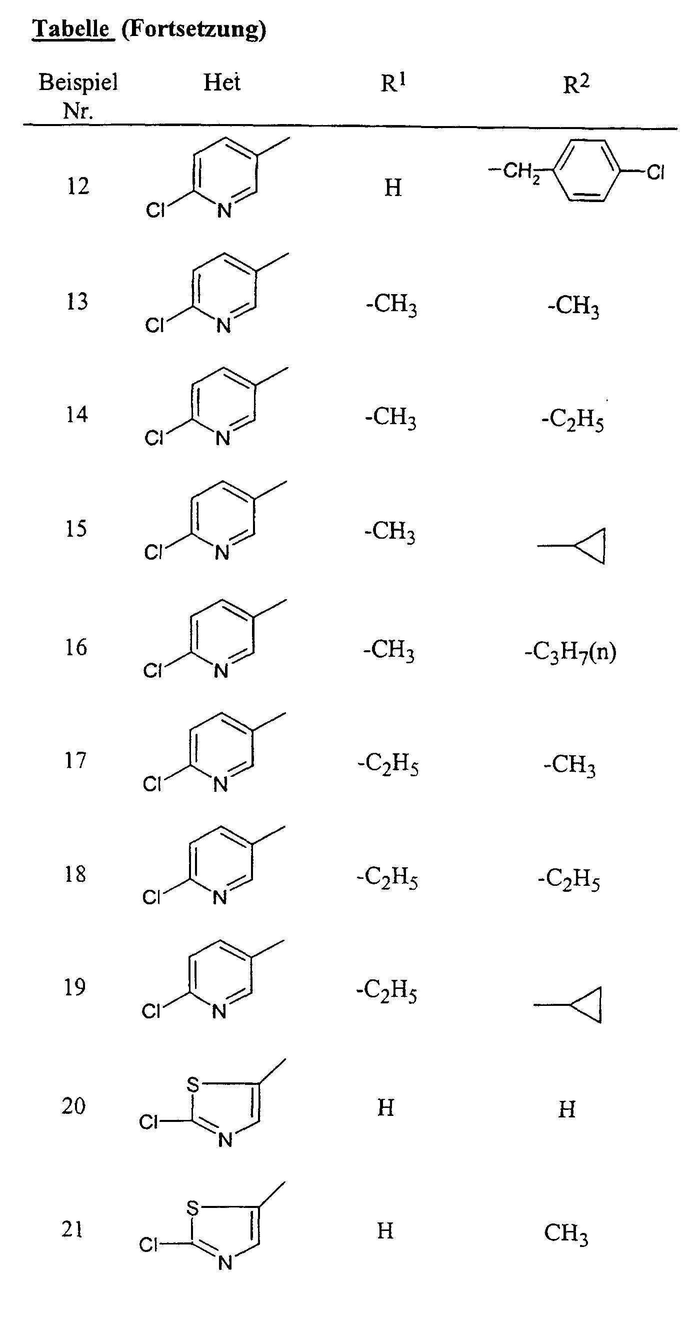

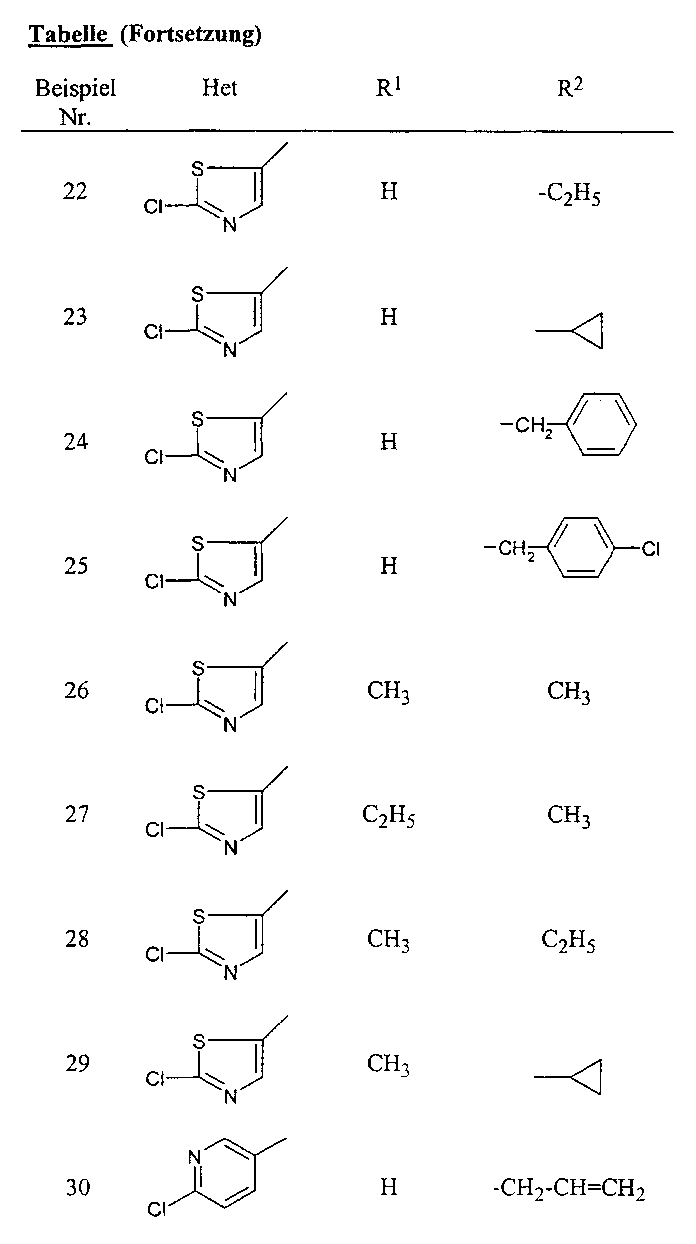

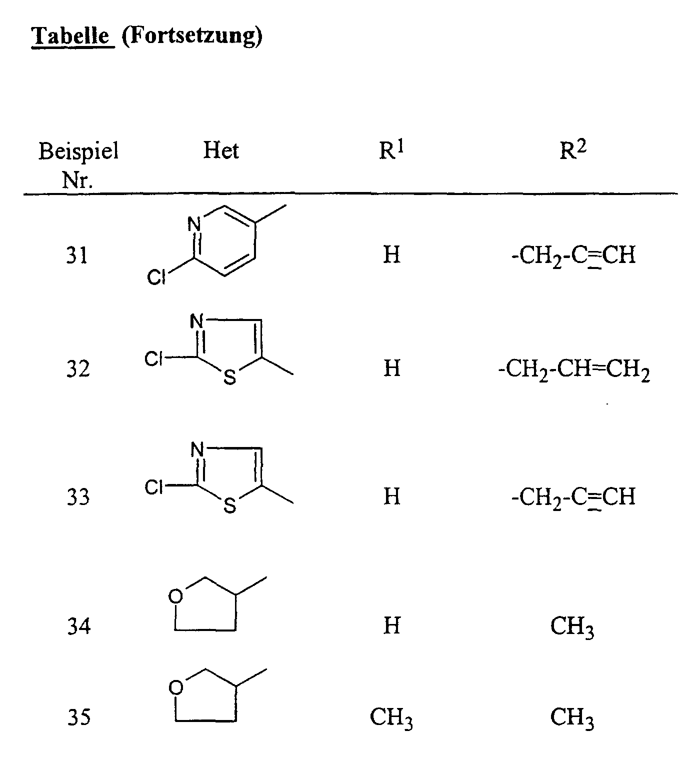

## Patentansprüche

1. Verfahren zur Herstellung von Verbindungen der Formel (I) 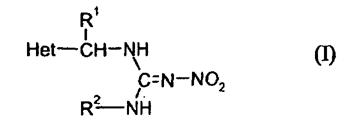 worin
R¹ für Wasserstoff oder C₁-C₄-Alkyl steht,
R² für Wasserstoff, C₁-C₆-Alkyl, C₃-C₆-Cycloalkyl oder -CH₂R³ steht,
R³ für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, für durch ein bis zwei Substituenten aus der Gruppe C₁-C₃-Alkyl, C₁-C₃-Halogenalkyl mit 1 bis 7 Halogenatomen, Cyclopropyl, Halogencyclopropyl, C₂-C₃-Alkenyl, C₂-C₃-Alkinyl, C₁-C₃-Alkoxy, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Halogen, Cyano und Nitro substituiertes 5-Thiazolyl; oder für durch ein bis vier Reste aus der Gruppe C₁-C₃-Halogenalkyl mit 1 bis 7 Halogenatomen, Cyclopropyl, Halogencyclopropyl, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy und Halogen substituiertes 3-Pyridyl steht,
Het für einen unsubstituierten oder substituierten aromatischen oder nichtaromatischen, monocyclischen oder bicyclischen heterocyclischen Rest steht,
**dadurch gekennzeichnet, dass** man eine Verbindung der Formel (II) 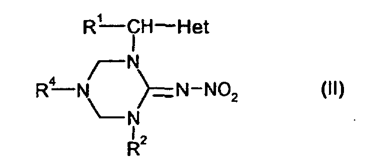 worin
R¹, R² und Het die oben angegebenen Bedeutungen haben und
R⁴ für C₁-C₁₀-Alkyl, C₃-C₆-Cycloalkyl, durch 1 bis 6 Reste aus der Gruppe Halogen, Hydroxy, C₁-C₄-Alkoxy, C₁-C₄-Halogenalkoxy mit 1 bis 9 Halogenatomen, Di-(C₁-C₄-Alkyl)-amino und C₁-C₅-Alkoxycarbonyl substituiertes C₁-C₁₀-Alkyl, durch 1 bis 4 Reste aus der Reihe C₁-C₄-Alkyl und Halogen substituiertes C₃-C₆-Cycloalkyl, Phenyl, Benzyl, oder durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro oder Cyano substituiertes Phenyl oder Benzyl, oder Heterocyclylmethyl steht, wobei Heterocyclyl für einen ungesättigten oder gesättigten 5- oder 6-gliedrigen Heterocyclus mit einem oder zwei Heteroatomen aus der Reihe Stickstoff, Sauerstoff, Schwefel steht,
mit wasserfreiem Chlorwasserstoff gegebenenfalls in Gegenwart eines Verdünnungsmittels oder mit einer oder mehreren Verbindungen, die mit protischen Lösungsmitteln Chlorwasserstoff entwickeln können umsetzt,
wobei die Reaktion wasserfrei durchgeführt wird.

2. Verfahren gemäß Anspruch 1, **dadurch gekennzeichnet, dass** man eine Verbindung der Formel (II) 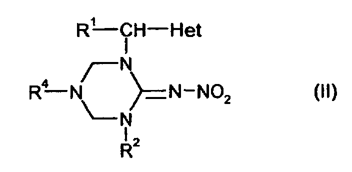 worin
R¹ für Wasserstoff oder C₁-C₄-Alkyl steht,
R² für Wasserstoff, C₁-C₆ Alkyl, C₃-C₆-Cycloalkyl oder -CH₂R³ steht,
R³ für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyndyl, 5-Thiazolyl, für durch einen Substituenten aus der Gruppe C₁-C₃-Alkyl, C₁-C₃-Halogenalkyl mit 1 bis 7 Halogenatomen, Cyclopropyl, Halogencyclopropyl, C₂-C₃-Alkenyl, C₂-C₃-Alkinyl, C₁-C₃-Alkoxy, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Halogen, Cyano und Nitro substituiertes 5-Thiazolyl; oder für durch ein oder zwei Reste aus der Gruppe C₁-C₃-Halogenalkyl mit 1 bis 7 Halogenatomen, Cyclopropyl, Halogencyclopropyl, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy und Halogen substituiertes 3 Pyridyl steht,
Het für einen unsubstituierten oder substituierten aromatischen oder nichtaromatischen heterocyclischen Rest aus der Reihe 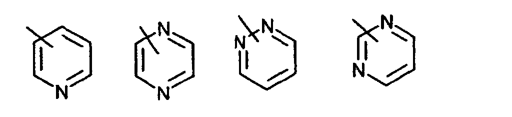 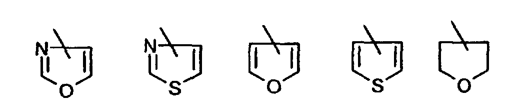 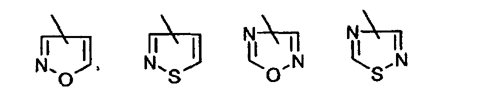 steht, der, auch in Abhängigkeit von der Art des Heterocyclus, ein oder zwei Substituenten aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy und Halogen enthalten kann,
R⁴ für C₁-C₁₀-Alkyl, C₃-C₆-Cycloalkyl, durch 1 bis 6 Reste aus der Gruppe Halogen, Hydroxy, C₁-C₄-Alkoxy, C₁-C₄-Halogenalkoxy mit 1 bis 9 Halogenatomen, Di-(C₁-C₄-Alkyl)-amino und C₁-C₅-Alkoxycarbonyl substituiertes C₁-C₁₀-Alkyl, durch 1 bis 4 Reste aus der Reihe C₁-C₄-Alkyl und Halogen substituiertes C₃-C₆-Cycloalkyl, Phenyl, Benzyl, oder durch 1 bis 3 Rmgsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro oder Cyano substituiertes Phenyl oder Benzyl, oder Heterocyclylmethyl steht, wobei Heterocyclyl für einen Heterocyclus aus der Reihe Furyl, Tetrahydrofuryl, Thienyl oder Pyridyl steht,
einsetzt.

3. Verfahren gemäß Anspruch 1 oder 2, **dadurch gekennzeichnet, dass** die Verbindungen, die mit protischen Lösungsmitteln Chlorwasserstoff entwickeln können ausgewählt sind aus Verbindungen der Formel (III) (Gruppe A): 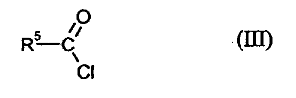 worin
R⁵ für C₁-C₁₀-Alkyl, C₃-C₁₀-Cycloalkyl, Aryl-C₁-C₄-alkyl steht, jeweils gegebenenfalls einfach oder mehrfach substituiert, wobei als Substituenten OH, SH, Halogen, C₁-C₆-Alkoxy, C₁-C₆-Alkylthio, C₁-C₆-Alkyl und Aryl, in Frage kommen, oder für Heterocyclylmethyl steht, wobei Heterocyclyl für einen ungesättigten oder gesättigten 5- oder 6-gliedrigen Heterocyclus mit einem oder zwei Heteroatomen aus der Reihe Stickstoff, Sauerstoff, Schwefel steht,
oder aus Verbindungen der Gruppe B:
reaktive Nichtmetall- und Metallchloride sowie reaktive Nichtmetall- und Metalloxychloride.

4. Verfahren gemäß Anspruch 3, **dadurch gekennzeichnet, dass** die Verbindungen, die mit protischen Lösungsmitteln Chlorwasserstoff entwickeln können ausgewählt sind aus Verbindungen der Formel (III) (Gruppe A): 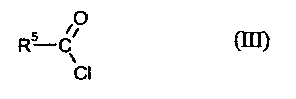 worin
R⁵ für C₁-C₁₀-Alkyl, C₃-C₁₀-Cycloalkyl, Phenyl-C₁-C₄-alkyl steht, jeweils gegebenenfalls einfach oder mehrfach substituiert, wobei als Substituenten OH, SH, Halogen, C₁-C₆-Alkoxy, C₁-C₆-Alkylthio, C₁-C₆-Alkyl und Phenyl in Frage kommen, oder für Heterocyclylmethyl steht, wobei Heterocyclyl für einen Heterocyclus aus der Reihe Furyl, Tetrahydrofuryl, Thienyl oder Pyndyl steht,
oder aus Verbindungen der Gruppe B:
Aluminiumchlorid, Phosphortrichlorid, Phosphorpentachlond, Thionylchlond, Sulfurylchlorid und Oxalylchlorid.

5. Verfahren gemäß Anspruch 1, 2, 3 oder 4, **dadurch gekennzeichnet, dass** Verbindungen der Gruppe A oder B, die mit protischen Lösungsmitteln Chlorwasserstoff entwickeln können, und polare protische Lösungsmittel verwendet werden.

6. Verfahren gemäß Anspruch 5, **dadurch gekennzeichnet, dass** als Lösungsmittel Alkohole oder Carbonsäuren verwendet werden.

7. Verfahren gemäß Anspruch 5 oder 6, **dadurch gekennzeichnet, dass** als Lösungsmittel Methanol, Ethanol, n-Propanol, i-Propanol, n-Butanol oder i-Butanol verwendet wird.

8. Verfahren gemäß Anspruch 1 oder 2, **dadurch gekennzeichnet, dass** wasserfreier Chlorwasserstoff und polare protische, polare aprotische oder unpolare, aprotische Lösungsmittel verwendet werden.

9. Verfahren gemäß Anspruch 8, **dadurch gekennzeichnet, dass** das Lösungmittel aus der Reihe Methanol, Ethanol, n-Propanol, i-Propanol, n-Butanol, i-Butanol, Aceton, Acetonitril, Essigsäureethylester, Diethylether, Diisobutylether, THF, Dioxan, Benzol, Toluol, Xylol, Methylenchlorid, Chloroform, Tetrachlorkohlenstoff, Chlorbenzol oder o-Dichlorbenzol ausgewählt ist.

## Claims

1. Process for the preparation of compounds of the formula (I) 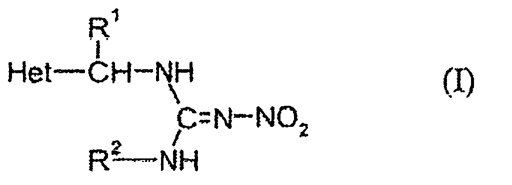 in which
R¹ is hydrogen or C₁-C₄-alkyl,
R² is hydrogen, C₁-C₆-alkyl, C₃-C₆-cycloalkyl or -CH₂R³,
R³ is C₂-C₅-alkenyl, C₂-C₅-alkynyl, phenyl, cyanophenyl, nitrophenyl, halophenyl having 1 to 3 halogen atoms, phenyl substituted by C₁-C₃-alkyl, C₁-C₃-haloalkyl having 1 to 7 halogen atoms, C₁-C₃-alkoxy or C₁-C₃-haloalkoxy having 1 to 7 halogen atoms, 3-pyridyl, 5-thiazolyl, is 5-thiazolyl substituted by one to two substituents from the group C₁-C₃-alkyl, C₁-C₃-haloalkyl having 1 to 7 halogen atoms, cyclopropyl, halocyclopropyl, C₂-C₃-alkenyl, C₂-C₃-alkynyl, C₁-C₃-alkoxy, C₂-C₃-haloalkenyl having 1 to 4 halogen atoms, C₂-C₃-haloalkynyl having 1 to 3 halogen atoms, C₁-C₃-haloalkoxy having 1 to 7 halogen atoms, C₁-C₃-alkylthio, C₁-C₃-haloalkylthio having 1 to 7 halogen atoms, allyloxy, propargyloxy, allythio, propargylthio, haloallyloxy, haloallylthio, halogen, cyano and nitro; or is 3-pyridyl substituted by one to four radicals from the group C₁-C₃-haloalkyl having 1 to 7 halogen atoms, cyclopropyl, halocyclopropyl, C₂-C₃-alkenyl, C₂-C₃-alkynyl, C₂-C₃-haloalkenyl having 1 to 4 halogen atoms, C₂-C₃-haloalkynyl having 1 to 3 halogen atoms, C₁-C₃-haloalkoxy having 1 to 7 halogen atoms, C₁-C₃-alkylthio, C₁-C₃-haloalkylthio having 1 to 7 halogen atoms, allyloxy, propargyloxy, allylthio, propargylthio, haloallyloxy, haloallylthio, cyano, nitro, C₁-C₃-alkyl, C₁-C₃-alkoxy and halogen,
Het is an unsubstituted or substituted aromatic or nonaromatic, monocyclic or bicyclic heterocyclic radical,
**characterized in that** a compound of the formula (II) 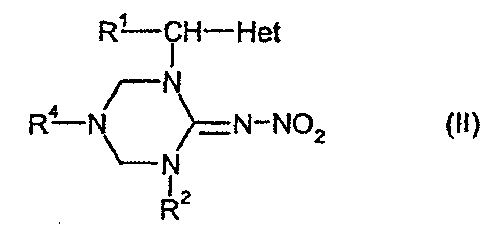 in which
R¹, R² and Het have the meanings given above and
R⁴ is C₁-C₁₀-alkyl, C₃-C₆-cycloalkyl, C₁-C₁₀-alkyl substituted by 1 to 6 radicals from the group halogen, hydroxyl, C₁-C₄-alkoxy, C₁-C₄-haloalkoxy having 1 to 9 halogen atoms, di(C₁-C₄-alkyl)amino and C₁-C₅-alkoxycarbonyl, C₃-C₆-cycloallcyl substituted by 1 to 4 radicals from the series C₁-C₄-alkyl and halogen, phenyl, benzyl, or phenyl or benzyl substituted by 1 to 3 ring substituents from the group halogen, C₁-C₄-alkyl, C₁-C₄-haloalkyl having 1 to 9 halogen atoms, C₁-C₄-alkoxy, C₁-C₄-haloalkoxy having 1 to 9 halogen atoms, C₁-C₄-alkylthio, nitro or cyano, or heterocyclylmethyl, where heterocyclyl is an unsaturated or saturated 5- or 6-membered heterocycle with one or two heteroatoms from the series nitrogen, oxygen, sulphur,
is reacted with anhydrous hydrogen chloride, if appropriate in the presence of a diluent, or with one or more compounds which can develop hydrogen chloride with protic solvents,
the reaction being carried out under anhydrous conditions.

2. Process according to Claim 1, **characterized in that** a compound of the formula (II) 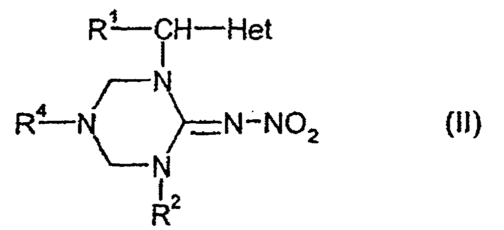 in which
R¹ is hydrogen or C₁-C₄-alkyl,
R² is hydrogen, C₁-C₆-alkyl, C₃-C₆-cycloalkyl or -CH₂R³,
R³ is C₂-C₅-alkenyl, C₂-C₅-alkynyl, phenyl, cyanophenyl, nitrophenyl, halophenyl having 1 to 3 halogen atoms, phenyl substituted by C₁-C₃-alkyl, C₁-C₃-haloalkyl having 1 to 7 halogen atoms, C₁-C₃-alkoxy or C₁-C₃-haloalkoxy having 1 to 7 halogen atoms, 3-pyridyl, 5-thiazolyl, is 5-thiazolyl substituted by one substituent from the group C₁-C₃-alkyl, C₁-C₃-haloalkyl having 1 to 7 halogen atoms, cyclopropyl, halocyclopropyl, C₂-C₃-alkenyl, C₂-C₃-alkynyl, C₁-C₃-alkoxy, C₂-C₃-haloalkenyl having 1 to 4 halogen atoms, C₂-C₃haloalkynyl having 1 to 3 halogen atoms, C₁-C₃-haloalkoxy having 1 to 7 halogen atoms, C₁-C₃-alkylthio, C₁-C₃-haloalkylthio having 1 to 7 halogen atoms, allyloxy, propargyloxy, allythio, propargylthio, haloallyloxy, haloallylthio, halogen, cyano and nitro; or is 3-pyridyl substituted by one or two radicals from the group C₁-C₃-haloalkyl having 1 to 7 halogen atoms, cyclopropyl, halocyclopropyl, C₂-C₃-alkenyl, C₂-C₃-alkynyl, C₂-C₃-haloalkenyl having 1 to 4 halogen atoms, C₂-C₃-haloalkynyl having 1 to 3 halogen atoms, C₁-C₃-haloalkoxy having 1 to 7 halogen atoms, C₁-C₃-alkylthio, C₁-C₃-haloalkylthio having 1 to 7 halogen atoms, allyloxy, propargyloxy, allylthio, propargylthio, haloallyloxy, haloallylthio, cyano, nitro, C₁-C₃-alkyl, C₁-C₃-alkoxy and halogen,
Het is an unsubstituted or substituted aromatic or nonaromatic heterocylic radical from the series 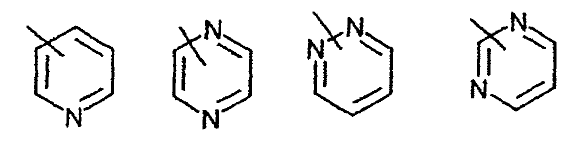 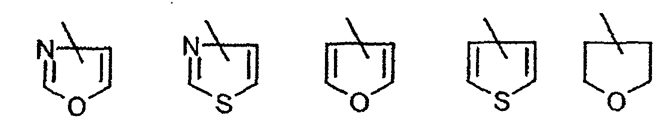 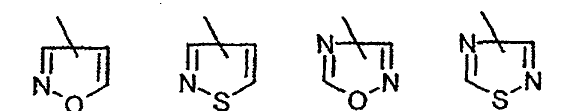 which, also depending on the type of heterocycle, can contain one or two substituents from the group C₁-C₃-haloalkyl having 1 to 7 halogen atoms, cyclopropyl, halocyclopropyl having 1 to 3 halogen atoms, C₂-C₃-alkenyl, C₂-C₃-alkynyl, C₂-C₃-haloalkenyl having 1 to 4 halogen atoms, C₂-C₃-haloalkynyl having 1 to 3 halogen atoms, C₁-C₃-haloalkoxy having 1 to 7 halogen atoms, C₁-C₃-alkylthio, C₁-C₃-haloalkylthio having 1 to 7 halogen atoms, allyloxy, propargyloxy, allylthio, propargylthio, haloallyloxy, haloallylthio, cyano, nitro, C₁-C₃-alkyl, C₁-C₃-alkoxy and halogen,
R⁴ is C₁-C₁₀-alkyl, C₃-C₆-cycloalkyl, C₁-C₁₀-alkyl substituted by 1 to 6 radicals from the group halogen, hydroxyl, C₁-C₄-alkoxy, C₁-C₄-haloalkoxy having 1 to 9 halogen atoms, di(C₁-C₄-alkyl)amino and C₁-C₅-alkoxycarbonyl, C₃-C₆-cycloalkyl substituted by 1 to 4 radicals from the series C₁-C₄-alkyl and halogen, phenyl, benzyl, or phenyl or benzyl substituted by 1 to 3 ring substituents from the group halogen, C₁-C₄-alkyl, C₁-C₄-haloalkyl having 1 to 9 halogen atoms, C₁-C₄-alkoxy, C₁-C₄-haloalkoxy having 1 to 9 halogen atoms, C₁-C₄-alkylthio, nitro or cyano, or heterocyclylmethyl, where heterocyclyl is a heterocycle from the series furyl, tetrahydrofuryl, thienyl or pyridyl,
is used.

3. Process according to Claim 1 or 2, **characterized in that** the compounds which can develop hydrogen chloride with protic solvents are chosen from compounds of the formula (III) (group A): 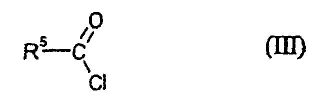 in which
R⁵ is C₁-C₁₀-alkyl, C₃-C₁₀-cycloalkyl, aryl-C₁-C₄-alkyl, in each case optionally monosubstituted or polysubstituted, suitable substituents being OH, SH, halogen, C₁-C₆-alkoxy, C₁-C₆-alkylthio, C₁-C₆-alkyl and aryl, or is heterocyclylmethyl, where heterocyclyl is an unsaturated or saturated 5- or 6-membered heterocycle with one or two heteroatoms from the series nitrogen, oxygen, sulphur,
or from compounds of group B:
reactive nonmetal and metal chlorides, and also reactive nonmetal and metal oxychlorides.

4. Process according to Claim 3, **characterized in that** the compounds which can develop hydrogen chloride with protic solvents are chosen from compounds of the formula (III) (group A): 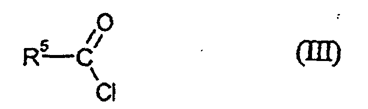 in which
R⁵ is C₁-C₁-alkyl, C₃-C₁₀-cycloalkyl, phenyl-C₁-C₄-alkyl, in each case optionally monosubstituted or polysubstituted, suitable substituents being OH, SH, halogen, C₁-C₆-alkoxy, C₁-C₆-alkylthio, C₁-C₆-alkyl and phenyl, or is heterocyclylmethyl, where heterocyclyl is a heterocycle from the series furyl, tetrahydrofuryl, thienyl or pyridyl,
or from compounds of group B:
aluminium chloride, phosphorus trichloride, phosphorus pentachloride, thionyl chloride, sulphuryl chloride and oxalyl chloride.

5. Process according to Claim 1, 2, 3 or 4, **characterized in that** compounds of group A or B which can develop hydrogen chloride with protic solvents, and polar protic solvents are used.

6. Process according to Claim 5, **characterized in that** alcohols or carboxylic acids are used as solvent.

7. Process according to Claim 5 or 6, **characterized in that** methanol, ethanol, n-propanol, isopropanol, n-butanol or isobutanol is used as solvent.

8. Process according to Claim 1 or 2, **characterized in that** anhydrous hydrogen chloride and polar protic, polar aprotic or nonpolar, aprotic solvents are used.

9. Process according to Claim 8, **characterized in that** the solvent is chosen from the series methanol, ethanol, n-propanol, isopropanol, n-butanol, isobutanol, acetone, acetonitrile, ethyl acetate, diethyl ether, diisobutyl ether, THF, dioxane, benzene, toluene, xylene, methylene chloride, chloroform, carbon tetrachloride, chlorobenzene or o-dichlorobenzene.

## Revendications

1. Procédé de préparation de composés de formule (1) 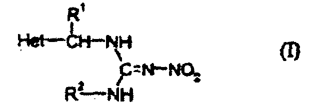 dans laquelle
R¹ désigne l'hydrogène ou un radical C₁-C₄-alkyle,
R² désigne l'hydrogène, un radical C₁-C₆-alkyle, C₃-C₆-cycloalkyle ou -CH₂R³,
R³ désigne un radical C₂-C₅-alcényle, C₂-C₅-alcinyle, phényle, cyanophényle, nitrophényle, halogénophényle avec 1 à 3 atomes d'halogène, un radical phényle substitué par un radical C₁-C₃-alkyle, C₁-C₃-halogénalkyle avec 1 à 7 atomes d'halogène, par un radical C₁-C₃-alcoxy ou C₁-C₃-halogénalcoxy avec 1 à 7 atomes d'halogène, désigne un radical 3-pyridyle, 5-thiazolyle, un radical 5-thiazolyle substitué par un à deux substituants du groupe C₁-C₃-alkyle, C₁-C₃-halogénalkyle avec 1 à 7 atomes d'halogène, cyclopropyle, halogénocyclopropyle, C₂-C₃-alcényle, C₂-C₃-alcinyle, C₁-C₃-alcoxy, C₂-C₃-halogénalcényle avec 1 à 4 atomes d'halogène, C₂-C₃-halogénalcinyle avec 1 à 3 atomes d'halogène, C₁-C₃-halogénalcoxy avec 1 à 7 atomes d'halogène, C₁-C₃-alkylthio, C₁-C₃-halogénalkylthio avec 1 à 7 atomes d'halogène, allyloxy, propargyloxy, allylthio, propargylthio, halogénallyloxy, halogénallylthio, par un radical halogène, cyano et nitro; ou désigne un radical 3-pyridyle substitué par un à quatre résidus du groupe C₁-C₃-halogénalkyle avec 1 à 7 atomes d'halogène, cyclopropyle, halogénocyclopropyle, C₂-C₃-alcényle, C₂-C₃-alcinyle, C₂-C₃-halogénalcényle avec 1 à 4 atomes d'halogène, C₂-C₃ halogénalcinyle avec 1 à 3 atomes d'halogène, C₁-C₃-halogénalcoxy avec 1 à 7 atomes d'halogène, C₁-C₃-alkylthio, C₁-C₃-halogénalkylthio avec 1 à 7 atomes d'halogène, allyloxy, propargyloxy, allylthio, propargylthio, halogénallyloxy, halogénallylthio, cyano, nitro, C₁-C₃-alkyle, C₁-C₃-alcoxy et un halogène,
Het désigne un radical monocyclique, bicyclique ou hétérocyclique, aromatique ou non aromatique, substitué ou non substitué,
**caractérisé en ce que** l'on met en réaction un composé de formule (II) 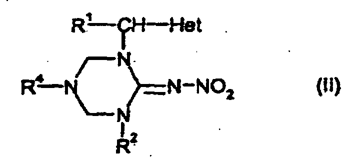 dans laquelle
R¹, R² et Het ont les significations précitées et
R⁴ désigne un radical C₁-C₁₀ alkyle, C₃-C₆-cycloalkyle, un radical C₁-C₁₀-alkyle substitué par 1 à 6 résidus du groupe halogène, hydroxy, C₁-C₄-alcoxy, C₁-C₄-halogénalcoxy avec 1 à 9 atomes d'halogène, di-(C₁-C₄-alkyl)-amino et C₁-C₅-alcoxycarbonyle, désigne un radical C₃-C₆-cycloalkyle substitué par 1 à 4 résidus de la série C₁-C₄-alkyle et un halogène, désigne un radical phényle, benzyle ou un radical phényle ou benzyle substitué par 1 à 3 substituants cycliques du groupe halogène, C₁-C₄-alkyle, C₁-C₄-halogénalkyle avec 1 à 9 atomes d'halogène, C₁-C₄-alcoxy, C₁-C₄-halogénalcoxy avec 1 à 9 atomes d'halogène, C₁-C₄-alkylthio, nitro ou cyano, ou désigne un radical hétérocyclylméthyle, le radical hétérocyclyle désignant un hétérocycle à 5 ou 6 éléments, saturé ou non saturé, avec un ou deux hétéroatomes de la série azote, oxygène et soufre,
avec du chlorure d'hydrogène anhydre, éventuellement en présence d'un agent de dilution ou avec un ou plusieurs composés qui peuvent former du chlorure d'hydrogène avec des solvants protiques,
la réaction se déroulant dans des conditions anhydres.

2. Procédé selon la revendication 1, **caractérisé en ce qu'**on utilise un composé de formule (II) 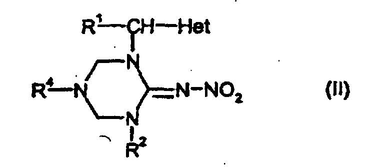 dans laquelle
R¹ désigne l'hydrogène ou un radical C₁-C₄-alkyle,
R² désigne l'hydrogène, un radical C₁-C₆-alkyle, C₃-C₆-cycloalkyle ou -CH₂R³,
R³ désigne un radical C₂-C₅-alcényle, C₂-C₅-alcinyle, phényle, cyanophényle, nitrophényle, halogénophényle avec 1 à 3 atomes d'halogène, désigne un radical phényle substitué par un radical C₁-C₃-alkyle, C₁-C₃-halogénalkyle avec 1 à 7 atomes d'halogène, par un radical C₁-C₃-alcoxy ou C₁-C₃-halogénalcoxy avec 1 à 7 atomes d'halogène, désigne un radical 3-pyridyle, 5-thiazolyle, un radical 5-thiazolyle substitué par un substituant du groupe C₁-C₃-alkyle, C₁-C₃-halogénalkyle avec 1 à 7 atomes d'halogène, cyclopropyle, halogénocyclopropyle, C₂-C₃-alcényle, C₂-C₃-alcinyle, C₁-C₃-alcoxy, C₂-C₃-halogénalcényle avec 1 à 4 atomes d'halogène, C₂-C₃-halogénalcinyle avec 1 à 3 atomes d'halogène, C₁-C₃-halogénalcoxy avec 1 à 7 atomes d'halogène, C₁-C₃-alkylthio, C₁-C₃-halogénalkylthio avec 1 à 7 atomes d'halogène, allyloxy, propargyloxy, allylthio, propargylthio, halogénallyloxy, halogénallylthio, par un radical halogène, cyano et nitro; ou désigne un radical 3-pyridyle substitué par un ou deux résidus du groupe C₁-C₃-halogénalkyle avec 1 à 7 atomes d'halogène, cyclopropyle, halogénocyclopropyle, C₂-C₃-alcényle, C₂-C₃-alcinyle, C₂-C₃-halogénalcényle avec 1 à 4 atomes d'halogène, C₂-C₃ halogénalcinyle avec 1 à 3 atomes d'halogène, C₁-C₃-halogénalcoxy avec 1 à 7 atomes d'halogène, C₁-C₃-alkylthio, C₁-C₃-halogénalkylthio avec 1 à 7 atomes d'halogène, allyloxy, propargyloxy, allylthio, propargylthio, halogénallyloxy, halogénallylthio, cyano, nitro, C₁-C₃-alkyle, C₁-C₃-alcoxy et un halogène,
Het désigne un radical hétérocyclique, aromatique ou non aromatique, substitué ou non substitué de la série 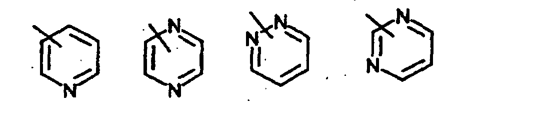 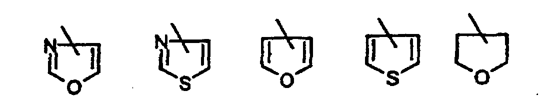 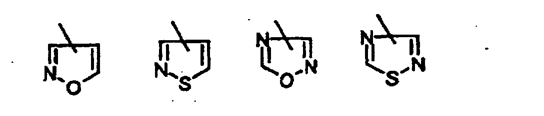 qui, en fonction de la nature de l'hétérocycle, peut contenir un ou deux substituants du groupe C₁-C₃-halogénalkyle avec 1 à 7 atomes d'halogène, cyclopropyle, halogénocyclopropyle avec 1 à 3 atomes d'halogène, C₂-C₃-alcényle, C₂-C₃-alcinyle, C₂-C₃-halogénalcényle avec 1 à 4 atomes d'halogène, C₂-C₃-halogénalcinyle avec 1 à 3 atomes d'halogène, C₁-C₃-halogénalcoxy avec 1 à 7 atomes d'halogène, C₁-C₃-alkylthio, C₁-C₃-halogénalkylthio avec 1 à 7 atomes d'halogène, allyloxy, propargyloxy, allylothio, propargylthio, halogénallyloxy, halogénallylthio, cyano, nitro, C₁-C₃-alkyle, C₁-C₃-alcoxy et un halogène,
R⁴ désigne un radical C₁-C₁₀ alkyle, C₃-C₆-cycloalkyle, un radical C₁-C₁₀-alkyle substitué par 1 à 6 résidus du groupe halogène, hydroxy, C₁-C₄-alcoxy, C₁-C₄-halogénalcoxy avec 1 à 9 atomes d'halogène, di-(C₁-C₄-alkyl)-amino et C₁-C₅-alcoxycarbonyle, un radical phényle ou benzyle substitué par 1 à 4 résidus de la série C₁-C₄-alkyle et C₂-C₆-cycloalkyle substitué par un halogène, désigne un radical phényle, benzyle ou un radical phényle ou benzyle substitué par 1 à 3 substituants cycliques du groupe halogène, C₁-C₄-alkyle, C₁-C₄-halogénalkyle avec 1 à 9 atomes d'halogène, C₁-C₄-alcoxy, C₁-C₄-halogénalcoxy avec 1 à 9 atomes d'halogène, C₁-C₄-alkylthio, nitro ou cyano, ou désigne un radical hétérocyclyl méthyle, l'hétérocyclyle désignant un hétérocycle de la série furyle, tétrahydrofuryle, thiényle ou pyridyle.

3. Procédé selon la revendication 1 ou 2, **caractérisé en ce que** les composés qui peuvent former du chlorure d'hydrogène avec des solvants protiques sont choisis parmi les composés de formule (III) (groupe A): 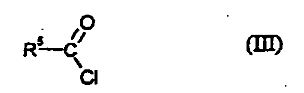 dans laquelle
R⁵ désigne un radical C₁-C₁₀-alkyle, C₃-C₁₀-cycloalkyle, aryl-C₁-C₄-alkyle, éventuellement substitués chacun une ou plusieurs fois, les radicaux OH, SH, halogène, C₁-C₆-alcoxy, C₁-C₆-alkylthio, C₁-C₆-alkyle et aryle peuvent être utilisés comme substituants, ou désigne un radical hétérocyclylméthyle, l'hétérocyclyle désignant un hétérocycle saturé ou non saturé à 5 ou 6 éléments avec un ou deux hétéroatomes de la série azote, oxygène et soufre,
ou des composés du groupe B:
chlorures réactifs métalliques et non métalliques ainsi qu'oxychlorures réactifs métalliques et non métalliques.

4. Procédé selon la revendication 3, **caractérisé en ce que** les composés qui peuvent former du chlorure d'hydrogène avec des solvants pratiques sont choisis parmi les composés de formule (III) (groupe A): 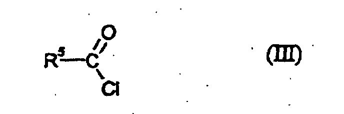 dans laquelle
R⁵ désigne un radical C₁-C₁₀-alkyle, C₃-C₁₀-cycloalkyle, phényl-C₁-C₄-alkyle, éventuellement substitués chacun une ou plusieurs fois, les radicaux OH, SH, halogène, C₁-C₆-alcoxy, C₁-C₆-alkylthio, C₁-C₆-alkyle et aryle pouvant être utilisés comme substituants, ou désigne un radical hétérocyclylméthyfe, l'hétérocyclyle désignant un hétérocycle de la série furyle, tétrahydrofuryle, thiényle ou pyridyle,
ou des composés du groupe B:
chlorure d'aluminium, trichlorure de phosphore, pentachlorure de phosphore, thionylchlorure, sulfurylchlorure et oxalylchlorure.

5. Procédé selon l'une des revendications 1, 2, 3 ou 4, **caractérisé en ce que** des composés des groupes A ou B qui peuvent former du chlorure d'hydrogène avec des solvants pratiques et des solvants pratiques polaires sont utilisés.

6. Procédé selon la revendication 5, **caractérisé en ce que** des alcools ou acides carboxyliques sont utilisés comme solvants.

7. Procédé selon la revendication 5 ou 6, **caractérisé en ce que** le méthanol, l'éthanol, le n-propanol, le i-propanol, le n-butanol ou le i-butanol sont utilisés comme solvant.

8. Procédé selon la revendication 1 ou 2, **caractérisé en ce que** du chlorure d'hydrogène anhydre et des solvants pratiques polaires, aprotiques polaires et aprotiques non polaires sont utilisés.

9. Procédé selon la revendication 8, **caractérisé en ce que** le solvant est choisi dans la série méthanol, éthanol, n-propanol, i-propanol, n-butanol, i-butanol, acétone, acétonitrile, éthylester d'acide acétique, diéthyléther, diisobutyléther, THF, dioxane, benzène, toluène, xylène, chlorure de méthylène, chloroforme, tétrachlorure de carbone, chlorobenzène ou o-dichlorobenzène.
